# EUROPEAN PATENT APPLICATION

(11) **EP 0 737 447 A1**
(43) Date of publication of application: **16.10.1996**
(21) Application number: 96302567.1
(22) Date of filing: 11.04.1996
(51) Int. Cl.: A61B 17/39

(54) **Electrosurgical hemostatic device with multiple selectable electrodes**

(30) Priority: 12.04.1995 US 421352
(71) Applicant: ETHICON ENDO-SURGERY, Cincinnati, Ohio 45242 (US)
(72) Inventor: Williamson IV, Warren P., Loveland, OH 45140 (US); Yates, David C., West Chester, OH 45069 (US)
(74) Representative: Fisher, Adrian John

(57) **Abstract**

An electrosurgical instrument is provided for cauterization and/or welding of tissue of varying impedances, thicknesses, vascularity and tissue treatment statuses, especially in the performance of endoscopic procedures. The instrument provides a tissue treating portion comprising at least three electrodes from which at least two electrode pairs of different electrical potentials of an electrosurgical system may be selected. The electrode pairs are strategically located with respect to the end effector, so that they may be selected to provide a desired result. They may also be selected based on a predetermined condition, for example, the impedance of the tissue as seen by the generator. Also, the tissue treatment locations may sequentially selected or the tissue temperature may be controlled. The electrode pair selection may be varied by an automated or user controlled switching device.

## Description

### Field of the Invention

This invention relates to an electrosurgical instrument for cauterization, coagulation and/or tissue welding in the performance of surgical procedures, especially endoscopic procedures.

### Background of the Invention

Surgical procedures requiring cutting of tissue can cause bleeding at the site of the cutting. Various techniques have been adapted to control bleeding with varying degrees of success such as, for example, suturing, applying clips to blood vessels, and stapling, as well as electrocautery and other tissue heating techniques. Advances in tissue joining or welding, tissue repair and wound closure also have permitted surgical procedures previously not possible or too risky.

Electrosurgical hemostatic devices have been described in various instruments. Most of the devices used are either monopolar or bipolar, for example, bipolar forceps, monopolar or bipolar scissors, and cutting and coagulating devices as described in EP-A-0640317 and EP-A-0640315.

When using electrosurgical energy, in electrosurgical devices, there may be an optimal range of tissue impedances that results in the best or optimal energy delivery for the output characteristics of the particular generator to which the impedance load is presented.

Generally, the optimal range is related to the principle that where the source and load impedances are matched, the transfer of power from the source to the load is maximized. Further, the power output for a given generator decreases at a predictable rate as impedance of the load, i.e., tissue, falls off of the source impedance.

The optimal range is defined herein as the range of load impedances at which the power transfer from the generator is sufficient to achieve the intended result, i.e., controlled coagulation, cauterization, or tissue welding. The optimal range may vary from application to application or from generator to generator.

It is believed that tissue impedance varies depending on a number of parameters which may include: tissue type, liquid content, tissue condition (i.e., coagulated or uncoagulated), tissue thickness, the amount of tissue compression, the size and length of the flow path of electrical current through the tissue, and energy frequency applied to tissue.

Additionally, for a given area or volume of tissue, the impedance model of the tissue is dynamic due to the fact that tissue impedance changes as tissue is heated and begins to coagulate, thus effecting the current flow pattern through the area or volume of tissue as coagulating current is delivered to the tissue. It is also believed that the tissue thickness typically changes as it is electrosurgically treated, because, for example, as water escapes in the form of steam or vapor the volume of the material grasped by the instrument is reduced. Depending on the specific end effector configuration, this could provide an additional variable in the impedance model of the tissue if in the particular end effector tissue thickness were to effect the length of the current path and/or the amount of compression applied to the tissue.

Thus, it is desirable to provide an electrosurgical device which can efficiently provide hemostasis in multiple tissue types and thicknesses, e.g., in fleshy or vascular tissue areas, and high, low or combination impedance tissues. Hemostasis is used herein, generally, to mean the arresting of bleeding including by coagulation, cauterization and/or tissue joining or welding.

It is also desirable to provide an instrument that brings a range of expected tissue impedance levels within an optimal load impedance range for the generator, and to obtain a desired result; e.g., controlled coagulation, cauterization, or tissue welding.

It is further desirable to provide a device which may be adapted to the changing impedance and/or tissue thicknesses as the tissue is being treated, so that the impedance presented to the generator is within an optimal range.

Another problem presented by presently known generators is that there may be a limitation to the amount of tissue that can be treated at one time. Thus, if it were to be desirable to coagulate a large area or volume of tissue with a single device, the device may be limited by the power delivery characteristics of the generator in use.

Thus, it is desirable to provide a device which will permit longer coagulation lines or greater coagulation areas without requiring excessive power.

In addition to meeting the generator load requirements for optimal energy delivery to tissue, it is also desirable to provide a device which can control the location of tissue treatment, coagulation, the resulting heat dissipation or thermal spread.

### Summary of the Invention

It is therefore an object of the invention to provide an electrosurgical method and device which optimize the efficiency of a tissue treating energy by providing electrodes which are selectable according to type, thickness, impedance, or other parameter of tissue to be engaged by or being treated by the end effector.

It is further an object of the invention to provide a device which is capable of sequentially firing electrodes over a length or area of tissue so that greater lengths or areas of tissue may be treated with an instrument at a time.

It is yet another object of the invention to provide selectable electrodes that may be used to control location, temperature or thermal spread of tissue being treated by an instrument

Accordingly, one embodiment of the present invention provides an adjustable device in which the distance between the opposite poles or electrodes of a tissue grasping or clamping device may be adjusted by selecting appropriate electrodes. The electrodes may be selected initially prior to treatment or during treatment to compensate for tissue thickness, impedance, various tissue types, or intended applications of the device.

In a preferred embodiment of the present invention an instrument adapts to the initial impedance or to the changing impedance as the tissue is being coagulated, so that energy is efficiently delivered to the tissue. The device may be continuously adjusted or may be adjusted at specific intervals during a period of treatment.

In a preferred embodiment the adjustment device includes an electrical switching device arranged to switch a potential between electrodes in different locations or between sets of opposite electrodes having different locations with respect to the end effector. The switching device may be arranged to switch between a number of permutations of electrode configurations at various locations on the device. The various locations may reflect, e.g., differences in distances between electrodes of different potentials, differences in locations with respect to the end effector, e.g., proximal or distal, within or without a zone of high compression. The switching may occur automatically or may be user controlled. Preferably the instrument includes a controller arranged to select an electrode pair based on the impedance of tissue and the load curve of the generator.

The electrodes or electrode sets may be switched to control or localize treatment of specific areas of the tissue. The electrodes are strategically placed on the end effector and the controller selects the electrodes for treatment based on electrode location or a parameter of tissue provided to the controller through a feedback mechanism.

Another embodiment of the invention provides an electrosurgical method and device which optimize the efficiency of tissue treating energy by providing segmented electrodes which may be sequentially fired to adapt to tissue type, length, area thickness, impedance, or other parameter of tissue to be engaged by the end effector. This allows the instrument to bring a range of expected tissue impedance level within an optimal load impedance range for the generator, so that the power requirements for the desired results, e.g., controlled coagulation, cauterization, or tissue welding, do not require power deliver capabilities that exceed that of the generator in use.

Another embodiment provides for tissue temperature control or control of thermal spread using strategically located electrode pairs and switching between the pairs. According to one aspect of this embodiment the electrodes in treatment system are configured and selected to direct the current in a desired path at a desired intensity for a desired duration of time. In one embodiment, electrode pairs are fired sequentially or in an alternating manner in response to feedback, thereby controlling the coagulation and heat dissipation throughout tissue.

In various embodiments of the invention, tissue condition feedback, e.g., tissue impedance, or other tissue parameters such as, infrared transmissivity, chemical composition, or tissue temperature, may be used to control the switching of electrode selection.

The device may be used in various instruments using therapeutic electrosurgical energy, such as, for example, tissue fastening devices, e.g. staplers, clip appliers, suturing devices, etc. Other end effector configurations may be used such as, for example, cutting devices, linear or circular cutting and stapling devices, etc.

These and other objects of the invention will be better understood from the following attached Detailed Description of the Drawings, when taken in conjunction with the Detailed Description of the invention.

### Detailed Description of the Drawings

Fig. 1 is a perspective view of an endoscopic electrosurgical clamping and coagulating device of one embodiment of the present invention;
Fig. 2 is a front cross sectional view of the distal end of the instrument of Fig. 1;
Fig. 3 is an exploded perspective view of the proximal handle portion of the instrument of Fig. 1;
Fig. 4 is an exploded perspective view of the intermediate and distal portion of the instrument of Fig. 1;
Fig. 5 is a side elevational view of the proximal handle portion in a first, open position of the instrument of Fig. 1, shown with the left side handle cover and wireforms removed;
Fig. 6 is an elevational view of the inside of the left side handle portion showing the location of the wireforms and connectors used in the present invention;
Fig. 7 is a longitudinal cross-sectional view of the intermediate portion of the instrument;
Fig. 8 is an elevational view of the proximal end of the intermediate portion showing the contact of the wireforms to their respective contact positions;
Fig. 9 is an enlarged cross-sectional view of the proximal end of the intermediate portion of the instrument;
Fig. 10 is a transverse cross sectional view taken along the lines 10-10 of Fig. 9;

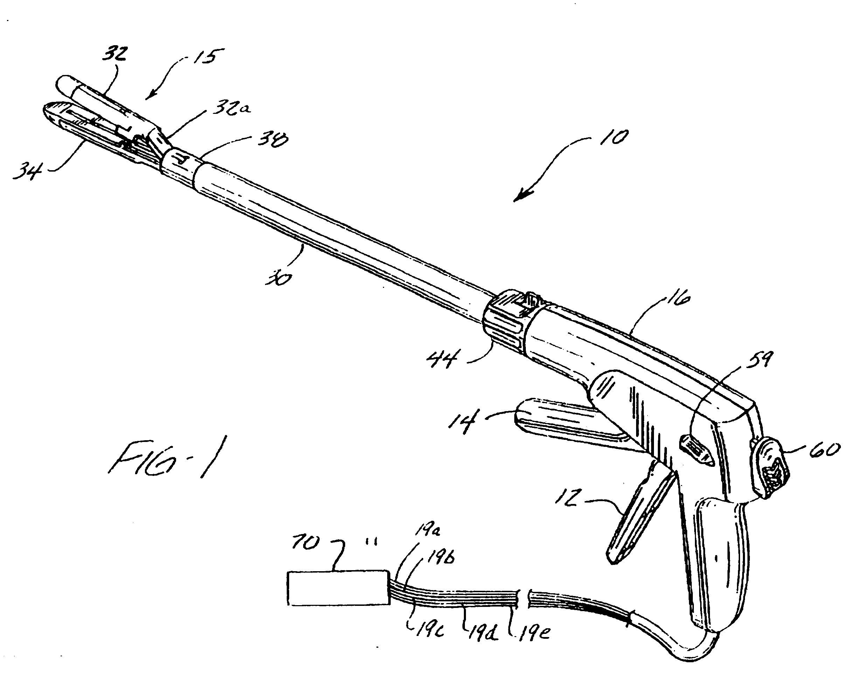

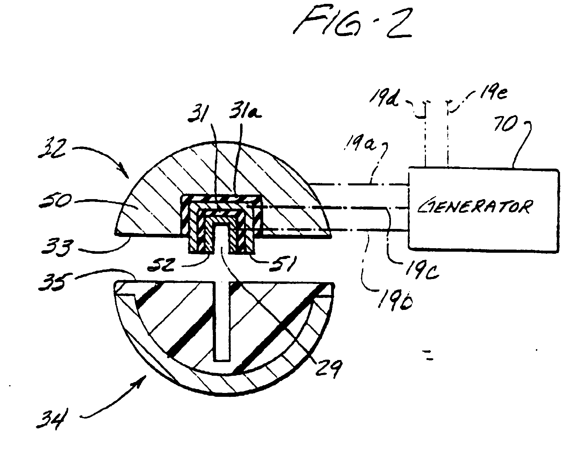

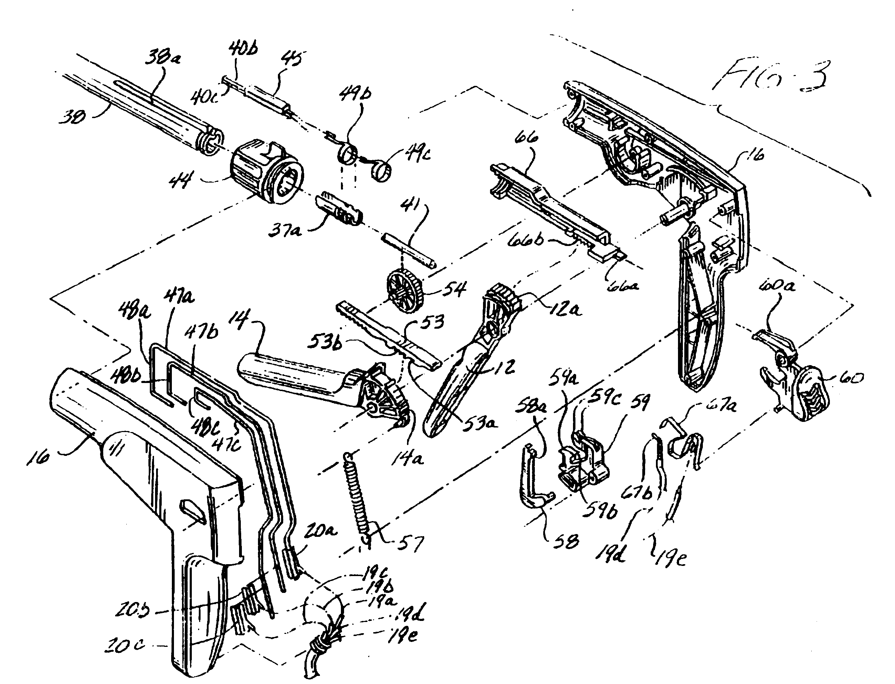

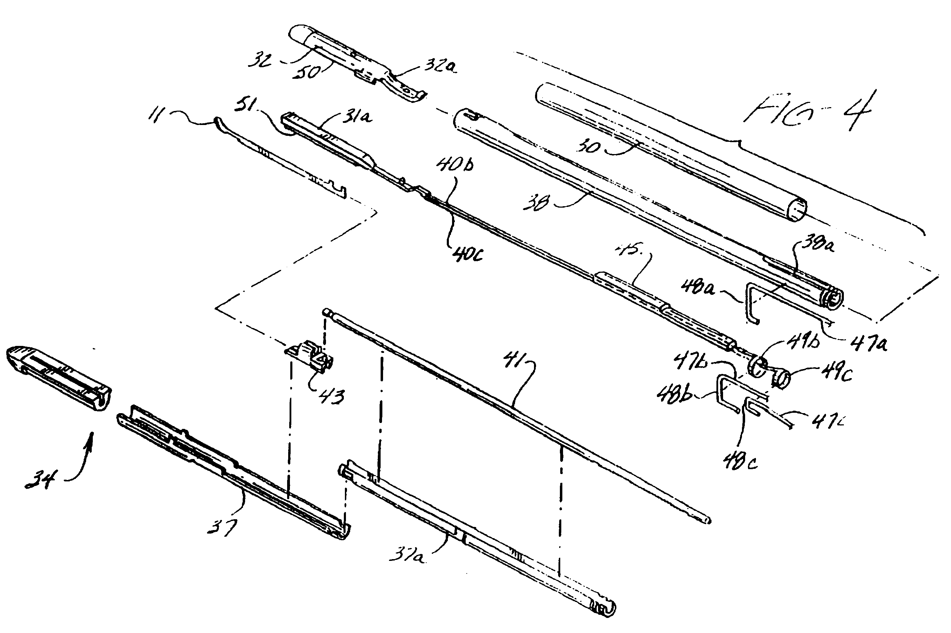

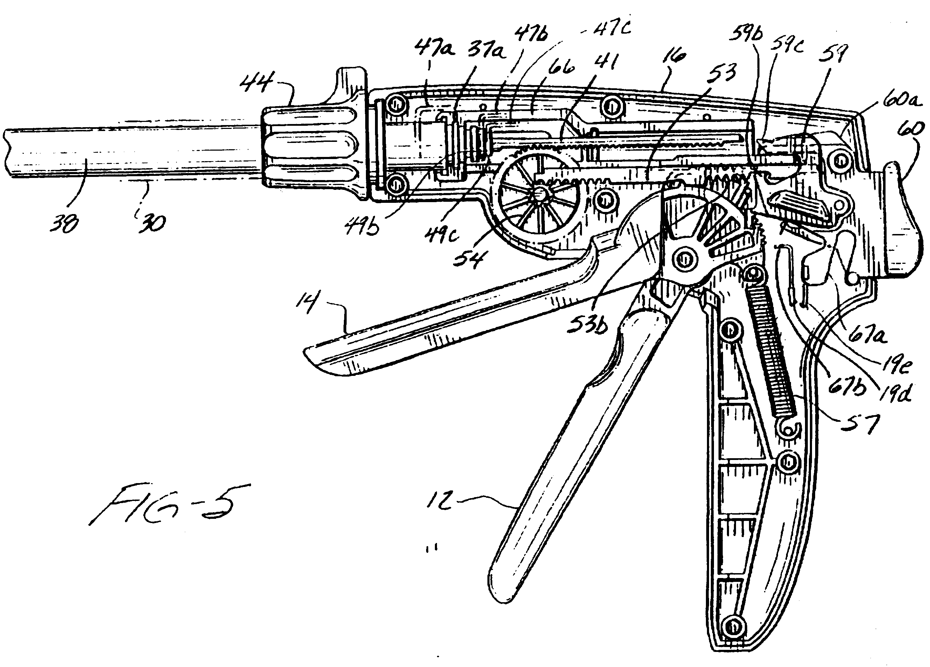

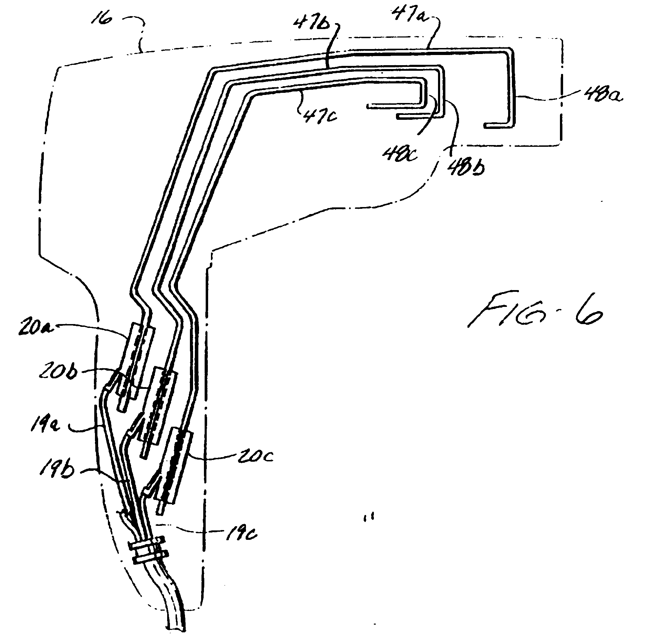

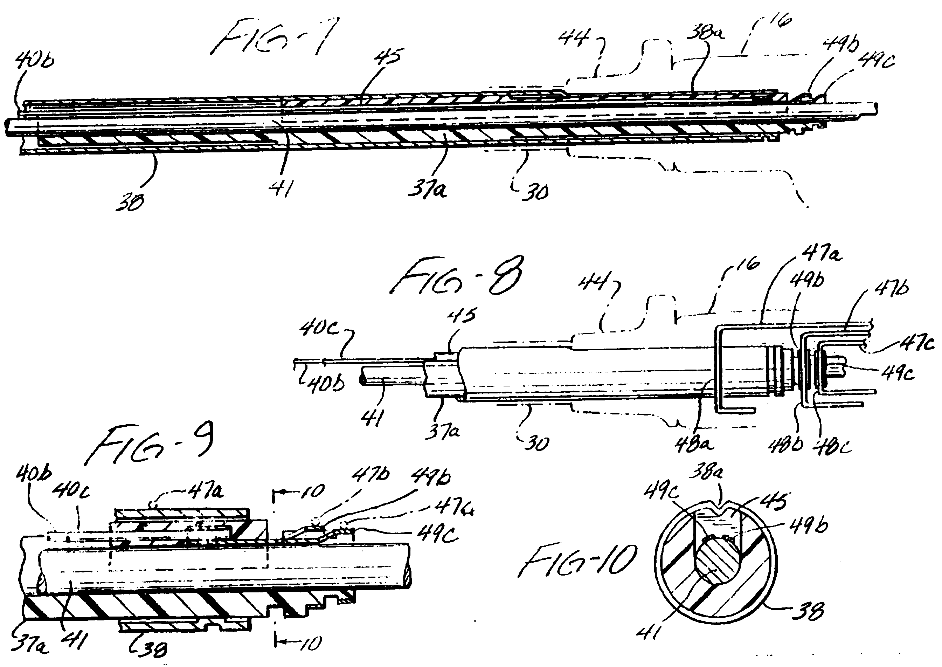

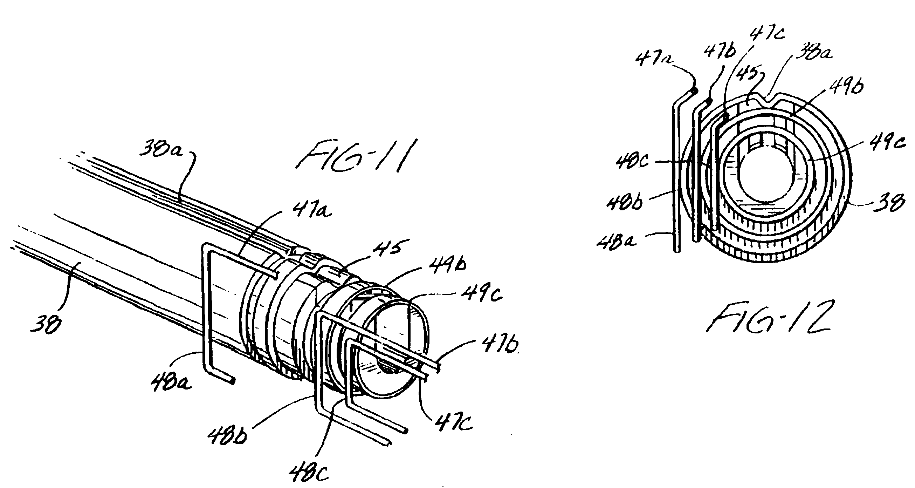

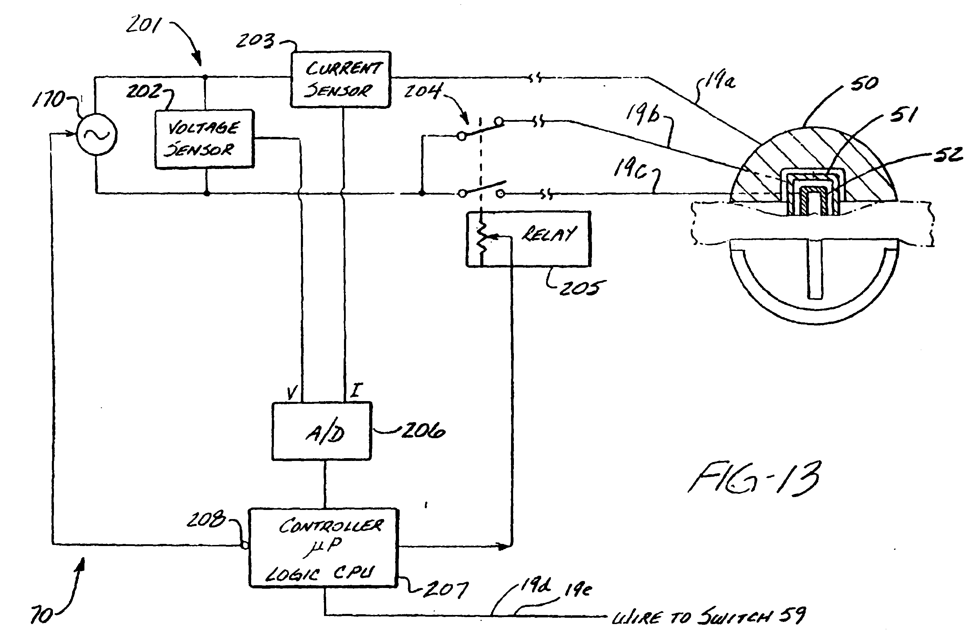

Fig. 1 1 is a perspective view showing the wireforms contacting their respective contact position;

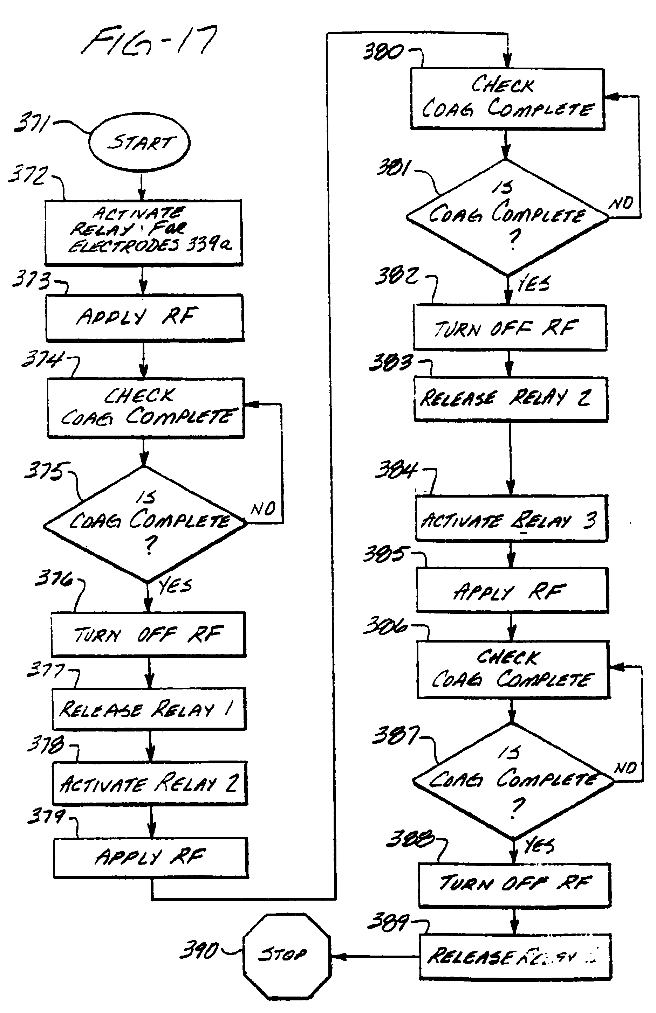

Fig. 12 is an end view of Fig. 11 showing a slight bias in the wireforms allowing for pressure of the wireforms onto their respective contact position; and
Fig. 13 is a block diagram of the switching mechanism of the device of Fig. 1;
Fig. 14 is a flow chart illustrating a preferred use of the instrument with an impedance feedback monitor;

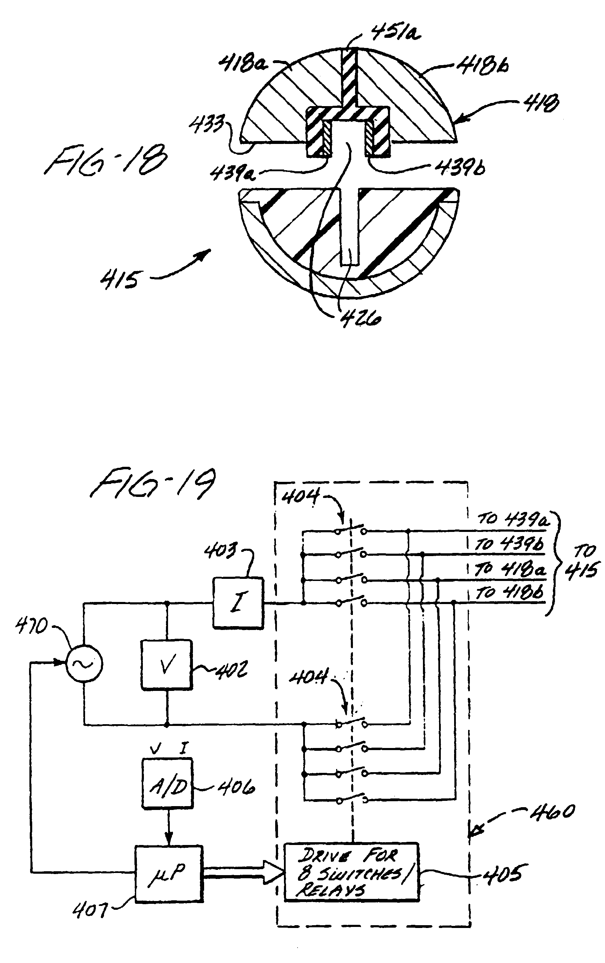

Fig. 15 is a perspective view of an end effector of an alternative embodiment;
Fig. 16 is a block diagram of a switch and control circuit in use with the instrument of Fig 15;
Fig. 17 is a flow chart illustrating operation of the device in Fig. 15;
Fig. 18 illustrates a front cross-sectional view of an end effector of an alternative embodiment;
Fig 19 is a flow chart illustrating operation of the device in Fig 18.

### Detailed Description of the Preferred Embodiments

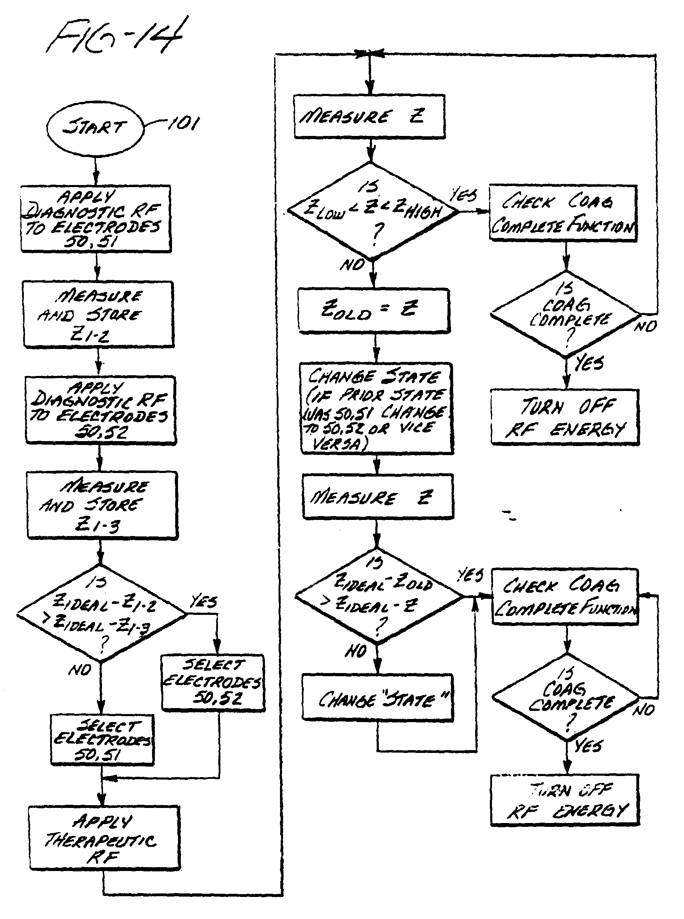

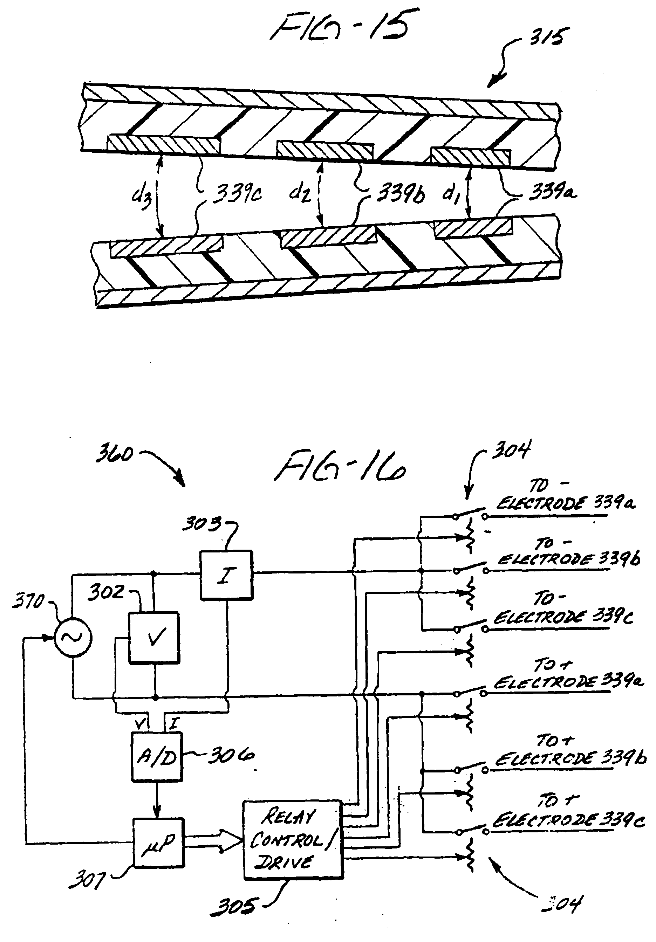

Referring now to Figs. 1-14 there is illustrated an instrument of preferred embodiment of the present invention.

An endoscopic clamping and cutting instrument 10 is shown having a housing 16 coupled to a sheath 30 with a lumen extending therethrough and an end effector 15 extending from the distal end of the sheath 30. The end effector 15 comprises first and second jaw members 32, 34. Jaw member 32 is movably secured to jaw member 34. The housing 16 has a clamping trigger 12 for closing jaw members 32, 34, an RF switch detente arm 58 and electrical switch contacts 67a, 67b, coupled to an electrical switch 59 for turning on RF energy, and a firing trigger 14 for advancing the cutting element 1 1 through tissue.

Jaw member 32 comprises a U-shaped electrode 52 forming a knife channel 29 extending longitudinally through jaw member 32. A U-shaped insulator 31 surrounds the electrode 52. U-shaped insulator 31 is surrounded by U-shaped electrode 51 which is in turn surrounded by a second insulator 31a. The insulators 31, 31a are preferably formed of a polymer such as polyphenyleneoxide. The remainder of the jaw member 32 formed of an electrically conductive material forming return electrode 50. The electrodes 50, 51, 52 are comprised of a conductor, such as, surgical grade stainless steel. Jaw member 32 has an inner surface 33 comprised of the electrodes 50, 51, 52 and insulators 31, 31a. Inner surface 33 substantially faces an inner surface 35 of jaw member 34.

In a preferred embodiment the electrodes 51 or 52 act as a first pole of a bipolar tissue treatment or therapeutic system depending on which of the two electrodes is selected. When the jaws 32, 34 are closed together the electrodes 50, 51, 52 are electrically isolated from each other by the insulators 31, 31a. The electrode 50 acts as a second therapeutic electrode of the bipolar treatment or therapeutic system.

In a preferred embodiment the device is used for a relatively thin, more compressible, or fatty tissue such as mesentery tissue or a relatively thick, more dense tissue such as muscular tissue or adnexa. Generally, fatty tissue tends to have a higher impedance, lower density and higher compressibility than muscular tissue or adnexa. The electrodes are arranged on interfacing surfaces to adapt to the expected tissue type. When thinner mesentery tissue is engaged electrode 51 may be selected as the first electrode so that the length of the current path is shorter thereby decreasing the relative impedance at the electrodes. Whereas, when thicker muscular or adnexa tissue is engaged, electrode 52 may be selected so that the length of the current path is longer thereby increasing the relative impedance as presented to the electrodes and thus the generator. Thus, in this embodiment, the appropriate selection of an electrode pair decreases the differences in load impedance between two different tissue types. The electrodes 50, 51, 52 may also be arranged so that electrodes 52, 51 are selected as opposite poles thus limiting the treatment to the center of the device.

The switching between electrodes may be user controlled or may be automatically selected by a controller. The controller may select the electrode based on a number of parameters, e.g. initial impedance values, tissue impedance feedback during the course of electrosurgical treatment, or other parameters indicating tissue parameters, tissue type or treatment status, e.g., coagulation status such as, e.g., tissue temperature. In the preferred embodiment set forth below, the electrodes are selected based on tissue impedance.

The sheath 30 is formed of an insulative material and has an electrically conductive closure tube 38 extending through its lumen. A channel retainer 37a extends from the proximal end of the closure tube 38 and is secured to channel 37 which there extends distally through the remainder of the closure tube 38 to form jaw member 34. The channel 37 includes jaw member 34 extending distally from the closure tube 38.

The body 16 has a clamping trigger 12 for advancing the closure tube 38 to close the jaws 32, 34 towards each other engaging tissue therebetween. Rotation of the clamping trigger 1 2 causes the closure tube 38 to advance co-axially through the sheath 30 over a camming surface 32a of jaw 32 to close the jaws 32, 34 onto tissue situated between the jaws 32, 34.

The channel retainer 37a guides co-axial movement of a drive rod 41 within the channel 37. The drive rod 41 is advanced by the rotation of the firing trigger 14 as described in more detail below. The driving rod 41 is coupled on its distal end to a block 43. The block 43 is coupled to a cutting means 1 which the drive rod 41 advances by way of the block 43 into the end effector 15. Jaw member 32 is secured by way of the channel 37 to the jaw member 34.

When the drive rod 41 advances the cutting element 11, the cutting element 11 advances through the knife channel 29 to cut tissue engaged by jaws 32, 34. This preferably occurs when the tissue has been electrosurgically treated. Thus, the cut line is medial to the coagulation lines formed by the bar electrodes 51 or 52.

A knob 44 located on the distal end of the body 16 rotates the closure tube 38, channel retainer 37a, channel 37 and end effector 15 which are directly or indirectly coupled to the knob 44 so that the knob 44 may be used for rotational placement of the end effector jaws 32, 34. The knob 44 includes a peg (not shown) which fits into and engages indentation 38a in closure tube 38. Closure tube 38 is fitted at its proximal end, into the housing 16.

Electrical energy is supplied to the electrodes 50, 51 and 52, by generator 70 through connections such as those described below, or other connections means, such as, for example, like those described in parent application S.N. 08/095,797, incorporated herein by reference. The generator 70 is user controlled by way of RF switch 59 located in the housing 16. Wires 19d, 19e extend from switch 59 to a controller included with the generator 70 as described below with respect to Fig. 13.

Wires 19a, 19b and 19c extend into the body 16 of the instrument and deliver energy to electrodes 50, 51 and 52 respectively. Wires 19a, 19b and 19c are coupled to low impedance contact elements 20a, 20b and 20c respectively and contact elements 20a, 20b and 20c are coupled to wireforms 47a, 47b and 47c respectively. Wireforms 47a, 47b and 47c are exposed at their distal ends 48a, 48b and 48c. Wireforms 47a, 47b and 47c are biased respectively towards closure tube 38, contact ring 49b and contact ring 49c located on the proximal end of channel retainer 37a, so as to make electrical contact with the contact closure tube 38, ring 49b and ring 49c respectively.

Wire 19a delivers electrical current to the electrode 50 by way of first wire form 47a which contacts electrically conductive closure tube 38 which contacts electrically conductive electrode 50 of jaw 32 as closure tube closes jaws.

Wire 19b delivers electrical current to the electrode 51 through second wire form 47b which contacts contact ring 49b coupled to wire 40b extending through the closure tube 38 to the electrode 51.

Wire 19c delivers electrical current to the electrode 52 through third wire form 47c which contacts contact ring 49c coupled to wire 40c extending through the closure tube 38 to the electrode 52.

The closure tube 38 and contact rings 49b, 49c permit the knob 44 to rotate while contact is maintained between closure tube 33, ring 49b and ring 49c, and wireforms 47a, 47b, 47c respectively. The closure tube 38, ring 49b and ring 49c are electrically insulated from each other.

Wires 40b, 40c extend through seal 45 which fits into channel retainer 37a, which fits into closure tube 38.

Clamping trigger 12 includes gear teeth 12a which movably engage with teeth 66b of yoke 66. Yoke 66 is coupled on its distal end to the closure tube 38. When clamping trigger 12 is actuated, the gear teeth 12a engage with teeth 66b in yoke 66 causing the yoke 66 to advance distally. Closure tube 38 closes jaws 32, 34 as it advances over camming surface 32a of jaw 32.

The RF switch 59 is rotated to switch on RF energy to be supplied to the therapeutic electrode pair 50, 51, or therapeutic electrode pair 50, 52 depending on which electrodes or pair is selected. When the RF switch 59 is rotated, detente protrusion 59a on the switch 59 hooks under detente protrusion 58a on detente arm 58, preventing the switch 59 from deactivating RF energy unless the RF switch 59 is manually rotated back to its original position. The RF energy may also be turned off electrically.

Switch 59 has a moveable contact 67a and a stationary contact 67b. The moveable contact 67a rotates with switch 59 to contact stationary contact 67b when switch is on. A signal is supplied to the generator 70 through wires 19d, 19e coupled to stationery contact 67b and moveable contact 67a respectively.

Ledge 60a of release button 60 is engaged with the proximal end of the yoke 66 adjacent step ledge 66a on proximal end of yoke 66. When the yoke 66 is advanced by the clamping trigger 12, the ledge 60a rotates down behind proximal end of yoke 66, thereby preventing yoke 66 from retracting until release button 60 has been pressed. Thus the jaws 32, 34 will remain in a closed position until a user releases the jaws 32, 34 with release button 60.

The switch 59 includes fingers 59c which sit just above proximal end of yoke 66. The ledge 60a of the release button 60 fits in between fingers 59c. The RF switch 59 cannot be activated, i.e., rotated forward, until the yoke 66 has been advanced distally so that fingers 59c of switch 59 are free to rotate behind proximal end of yoke 66.

The switch 59 also includes a lower hook 59b which engages groove 53a of firing rack 53. Firing rack 53 includes gear teeth 53b which are engaged by gear teeth 14a of firing trigger 14. The firing rack 53 is coupled on its distal end to pinion gear 54 which in turn engages the drive rod 41.

When the firing trigger 14 is pulled, the fire rack 53 is advanced distally to rotate pinion 54 which advances the driving rod 41 distally to actuate the cutting element 11 to cut tissue engaged by the end effector 15.

The firing rack 53 cannot advance however until the lower hook 59b of the RF switch is disengaged from the groove 53a of the firing rack 53. This occurs only when the RF switch 59 has been activated.

Thus, the presently described device includes a lockout device or devices for preventing application of RF energy, or knife actuation until the jaws 32, 34 have been closed. The lockout device(s) require the proper sequence is followed, i.e, jaw closure, followed by application of RF energy, followed by cutting element actuation. It also provides a detented RF switch so that RF energy is continuously applied until the switch 59 is manually released or until the RF energy is switched off, e.g., by an electrical feedback control signal to the generator 70.

The closure trigger 12 and firing trigger 14 are interlocked and a spring 57 is mechanically coupled to both triggers 12, 14.

When tissue is engaged between clamped jaw members 32, 34, and RF energy has been applied, the firing trigger 14 located on housing 16 may be actuated to advance a cutting element 11 through the engaged tissue to cut the tissue.

A preferred switching and control device is illustrated in Figs. 13 and 14. Fig. 13 illustrates a block diagram of a controller 207 in use with a switching mechanism of the present invention. A source 170 supplies energy through circuit 201 to an electrode pair comprising electrodes 50, and 51 or 52. Switch relay contacts 204 select which electrode 51 or 52, will be the first pole of a bipolar system. Electrode 50 is the second pole of the bipolar system. Switch relay contacts 204 are controlled by a relay control 205 which receives a switch control signal from the controller 207.

A voltage sensor 202 in parallel with the circuit 201 senses the voltage supplied to the tissue through the selected electrode pair and provides a signal proportional to the RF voltage. A current sensor 203 in series with the circuit 201 senses the current delivered to the tissue through the electrode pair and provides a signal proportional to the RF current. The voltage signal from the voltage sensor 202 and the current signal from the current sensor 203 are communicated to an A/D convertor 206. The A/D convertor 206 provides a representative voltage signal and current signal to the controller 207.

The voltage and current signals may be used by the controller 207 to compute a number of tissue characteristics. In this preferred embodiment, the voltage and current representative signals are used to compute the impedance of the tissue being treated by electrodes. Based on the impedance of the tissue, the controller provides a signal to the relay control 205 to select the appropriate electrode 51 or 52. The controller also controls the energy source 170 through control output 208. The controller may include a microprocessor (CPU) or other digital or analog circuitry.

In Fig. 14, a flow chart illustrates the use of the controller with the instrument. In block 101 the device is turned on and ready for use. The controller output 208 sends a signal to the source 170 to supply diagnostic RF energy to electrodes 50, 51 (block 102). The diagnostic RF energy is preferably at a lower voltage and/or different frequency than the therapeutic RF energy which will be delivered to tissue when tissue treatment begins. The impedance is determined and stored as the Z₁₋₂ using voltage and current signals from the voltage sensor 202, current sensor 203 and the A/D convertor 206 (block 103).

Then the controller 207 sends a signal to the relay control 205 to cause relay contacts 204 to toggle to electrodes 50, 52 and sends a control signal from control output 208 to tell the source 170 to apply diagnostic RF energy to electrodes 50, 52 (block 104). Using the signals from voltage sensor 202 and current sensor 203, impedance is determined by the controller 207 and stored as Z₁₋₃ (block 105).

A decision is made whether the first impedance, Z₁₋₂ or the second impedance, Z₁₋₃, provides the best impedance for the generator load characteristics (block 106). Thus a predetermined ideal impedance, Z_{ideal}, is compared to Z₁₋₂ and Z₁₋₃. If Z_{ideal} minus Z₁₋₂ is greater than Z_{ideal} minus Z₁₋₃ then electrodes 50, 52 are selected (block 107). If not, then electrodes 50, 51 are selected (block 108).

After the appropriate electrode pair has been initially selected according to blocks 102 through 108, the controller 207 sends a control signal from the output 208 to the source 170 to tell the source 170 to supply therapeutic RF energy through the selected electrode pair (block 109). As the therapeutic RF energy is applied, tissue impedance is measured in the same manner as set forth in blocks 103, 105 (block 110).

If the measured impedance Z is higher than a predetermined minimum impedance, Z_{low} and lower than a predetermined maximum impedance, Z_{high}, then the controller 207 moves to block 112 (block 111). In block 112 the controller 207 determines whether coagulation is complete. Various means of determining whether coagulation is complete based on impedance, voltage and/or current may be used. For example, one such coagulation complete checking function is set forth in U.S. Application Serial No. 08/311,297 incorporated herein by reference. If coagulation is complete (block 113), then RF energy is turned off (block 114). If coagulation is not complete then the controller 207 goes back to block 110 where the impedance is measured until coagulation is complete or the measured impedance is below Z_{low} or above Z_{high}.

If the impedance is below Z_{low} or above Z_{high}, the controller 207 considers whether or not to select alternate electrode pairs. First, a variable, Z_{old}, is set as the measured impedance (block 115). Then, the controller 207 sends a signal to a relay control 205 to change relay contacts 204 to select the alternate electrode pair (block 116). If the prior state was 50, 51 as the selected electrodes then the state is changed to select electrodes 50, 52, or visa versa.

The impedance is then measured using the newly selected electrode pair (block 117). The impedance at the first electrode pair, Z_{old}, is compared to the impedance at the newly selected pair as measured in block 117. Whichever impedance is closer to the ideal impedance for the generator, Z_{ideal}, the corresponding electrodes are selected for completion of the tissue treatment (block 118). If Z_{ideal} minus Z_{old} is greater than Z_{ideal} minus the measured impedance at the presently selected electrode pair, then the controller moves on to block 120 in which it continues to check coagulation complete function using the newly selected electrode pair until coagulation is complete (block 121), at which point the RF energy is turned off (block 122). If the electrode pair newly selected does not provide an impedance which is closer to the ideal impedance, Z_{ideal}, than Z_{old}, then the controller 207 causes the relay control 205 to switch back to the previously selected electrode pair (block 119).

In other embodiments, this state may be changed between the two electrodes on a continuous basis until coagulation is complete. In this preferred embodiment however, the electrode pair selected initially and therapeutic energy is applied until either coagulation is complete or the impedance is out of the ideal impedance range, at which point the electrodes may be switched. When the electrodes are switched a check is run to confirm that the appropriate electrode has been selected in switching the electrodes. In either case, in this particular embodiment, the electrode is only switched one more time and then the treatment is completed.

Referring now to Figs. 15-17 the end effector 315 of an instrument comprising segmented electrode pairs 339a, 339b, 339c is illustrated. 339a has a longer length and treats tissue closer to the proximal end of the end effector. Electrode pairs 339a are a distance, d₁, from each other and present a relatively lower impedance to the generator. Similarly, electrode pairs 339b have a shorter length than electrode 339a but has a greater distance, d₂, between electrode pairs 339b because of the relatively more distal location on the end effector. Finally, the distal most electrode pairs 339c, are shorter than the electrode pairs 339a, 339b and yet have a further distance, d₃, from each other because of the cantilevered beam arrangement of the end effector. This increases the impedance of the tissue at electrode 339c as compared to that at electrodes 339a and 339b effect. The impedance seen at pair 339a, Z1, is less than the impedance seen at pairs 339b, Z2, which is less than the impedance seen at pairs 339c, Z3.

Referring to Figs 16-17, a switching mechanism 360 is illustrated and described for use with the end effector of Fig. 15. A source 370 supplies an electrosurgical signal to a selected one of electrode pairs 339a, 339b or 339c. A voltage sensor 302 and current sensor 303 sense the voltage a current delivered by the source 370 and provide a corresponding signal to an A/D convertor 306. The A/D convertor 306 converts the signal to a digital signal which is provided to the controller 307. The controller 307 provides a relay control signal to the relay control 305 based on the measured tissue parameter of impedance. The re!ay control 305 drives the relay contacts 304 to select an appropriate electrode pair for delivery of electrosurgical energy to tissue. The controller 307 also provides a control signal to control energy source 370.

In use, the device is turned on (block 371) and the controller 307 activates the relay control 305 for electrode pair 339a (block 372). The controller 307 sends signal to the source 370 to apply RF energy (block 373). The voltage and current sensors 302, 303 send a representative voltage and current signal to the controller 307 by way of A/D converter 306 from which coagulation complete may be determined as described, for example in U.S. Application Serial No. 08/311,297 (blocks 374, 375.) When coagulation is complete, RF is turned off energy to electrode pair 339a (block 376, 377). The controller 307 then sends a signal to the relay control 305 to activate relay contact 304 corresponding to electrode pair 339b. (block 378) The controller than signals the source 370 to supply energy to the electrode pair 339b (block 379). Coagulation complete of tissue treated by electrode pair 339b is checked as described above with respect to electrode pair 339a and the relay control 305 is signaled to switch to electrode pair 339c when coagulation at pair 339b is complete (blocks 380-384) RF energy is supplied to electrode pair 339c until coagulation is complete there (blocks 385-390). Any electrode or electrode combinations may be selected in a similar manner in alternative embodiments of the invention.

Alternatively the electrodes may be fired in a predetermined sequence, e.g., proximal to distal. Each electrode is coupled to an impedance feedback monitor which is coupled to a controller. The impedance is monitored at the first fired electrode pair until coagulation is complete at the electrode. Then the controller selects the next electrode of the sequence, and so on, until coagulation is complete. An impedance feedback monitoring device which may be used is set forth in U.S. Application No. 08/311,297.

Referring now to Fig. 18 and 19 there is illustrated an additional embodiment of an end effector 415 of the device of the present invention. In this embodiment rather than the electrodes being segmented along the length of the end effector, electrodes 439a and 439b are located on opposite sides of knife channel 426. The return electrode, the anvil 418 is located on the same interfacing surface 433 as the electrodes 439a, 439b and is divided into two electrically isolated portions 418a, 418b which are separated by an insulating material 451a. Preferably, the switching mechanism 460 is arranged to switch between either electrode 439a or 439b which when selected forms a first pole of a bipolar system and either electrode 418a or 418b which when selected forms a second pole. The energy delivered to the tissue will be concentrated on one side of the end effector and then when switched on the other side of the end effector.

As illustrated in Fig. 19 any two or more of the electrodes may be selected to provide an electrode pair. One of six electrode pairs may be selected, 439a and 418a; 439a and 418b; 439a and 439b; 439b and 418a; 439b and 418b or 418a and 418b. The switching may occur rapidly so that heat will be concentrated along a center line of the grasped tissue between electrode pairs formed by two or more of electrodes 439a, 439b, 418a, and 418b. The switching device may also be adapted to switch so that electrode 439a corresponds to one pole of the bipolar system and electrode 439b corresponds to the second pole of the electrosurgical system and thus the electrosurgical energy delivery is limited to an area directly in the center of the device. The appropriate electrodes may be selected according to the intended affect. For example, the center of the tissue may be treated first employing electrodes 439a and 439b and then the sides of the tissue may be treated employing the anvil 418a or 418b and one or both of the segmented electrodes 439a, 439b.

As illustrated in Fig. 19, impedance may be used as a tissue parameter from which an electrode pair is selected by the controller 407. Voltage sensor 402 senses voltage and current sensor 403 senses current delivered by source 470 to tissue engaged by end effector 415. Signals proportional to current and voltage are supplied to an A/D converter 406 which provides a digital signal to controller 407. Based on the received voltage and/or current signals, the controller 407 provides a signal to relay control 405 which drives the appropriate relay contact 404 in response to relay control signal from the controller 407. The controller 407 also provides a control signal to energy source 470 which provides therapeutic and or query electrosurgical energy to a selected electrode pair.

Several variations of this invention have been described in connection with specific embodiments. Naturally, the invention may be used in numerous applications where hemostasis or other electrosurgical tissue effects are desired. For example, the devices described herein may include a mechanism for cutting tissue and/or applying staples or other fasteners. See for example EP-A-0640317 and EP-A-0640315, incorporated herein by reference. Accordingly, it will be understood by those skilled in the art that various changes and modifications may be made in the invention without departing from its scope, which is defined by the following claims and their equivalents.

## Claims

1. An electrosurgical instrument comprising:
a shaft; and
a tissue treating portion, said tissue treating portion comprising:
at least three electrodes electrically isolated from each other and arranged to receive and deliver electrosurgical energy therethrough,
a switching device operatively coupled to at least two of said at least three electrodes, said switching device capable of forming at least two electrode pairs, each of said at least two electrode pairs comprising two electrodes of different electrical potentials of a therapeutic electrosurgical system; and
a controller operatively coupled to said switching device, said controller including a tissue status measuring device, said tissue status measuring device arranged to provide tissue status information to the controller, and said controller arranged to provide a control signal to said switching device for selecting an electrode pair in response to said tissue status information.

2. The electrosurgical instrument of claim 1 wherein said tissue status information comprises an electrical parameter.

3. The electrosurgical instrument of claim 1 wherein said tissue status information comprises impedance of tissue being treated by said electrode pair.

4. The electrosurgical instrument of claim 1 said controller includes an impedance feedback monitor arranged to monitor impedance of tissue over a period of tissue treatment with said electrode pair, to determine when tissue has been treated to a desired degree based on said monitored impedance and to provide a treatment complete control signal upon the condition that said tissue has been treated to a desired degree by said electrode pair.

5. An electrosurgical device comprising:
an end effector comprising:
first and second opposing interfacing surfaces, said interfacing surfaces capable of engaging tissue therebetween;
a first electrode;
a second electrode; and
a third electrode, said electrodes electrically isolated from each other,
wherein said first electrode is located on at least one of said interfacing surfaces, said second electrode is located on at least one of said interfacing surfaces, and said third electrode is located on at least one of said interfacing surfaces,
said end effector arranged to receive therein, electrosurgical energy of a first electrical potential and of a second electrical potential different from the first potential, and to conduct the electrosurgical energy through at least two of said first, second and third electrodes; and
a switch operatively coupled to at least two of said first, second and third electrodes arranged to select a pair of said electrodes of electrically different electrical potentials to receive electrosurgical energy;
wherein said second electrode is located a first distance from said first electrode with respect to said at least one of said interfacing surfaces and wherein said third electrode is located a second distance from said first electrode with respect to said at least one of said interfacing surfaces.

6. The electrosurgical device of claim 5 wherein said switch is arranged to switch said first electrical potential between said second electrode and said third electrode.

7. The electrosurgical device of claim 6 wherein said switch is arranged to switch said first electrical potential between said second electrode and said third electrode as electrosurgical energy is applied to tissue.

8. The electrosurgical device of claim 5 wherein said switching device is arranged to select an electrode pair for electrosurgical tissue treatment according to a parameter of tissue engaged by said surface.

9. The electrosurgical device of claim 8 further comprising a tissue monitoring device operatively coupled to said end effector and said switch, wherein said tissue monitoring device is arranged to monitor the tissue parameter and control the switch to select the electrode pair according to said monitored parameter.

10. A surgical instrument comprising:
a shaft having a distal end; and
an end effector located at the distal end of said shaft said end effector comprising:
first and second elements comprised of first and second opposed tissue contacting surfaces respectively, said elements movable relative to each other from an open, spaced apart position for positioning tissue therebetween, to a closed position for approximating the tissue between the surfaces,
at least a portion of one of said tissue contacting surfaces comprising a first electrode, at least a portion of one of said tissue contacting surfaces comprising a second electrode, and at least a portion of one of said tissue contacting surfaces comprising a third electrode, wherein said first, second and third electrodes are electrically isolated from each other;
said end effector arranged to receive therein, electrosurgical energy of a first electrical potential and of a second electrical potential different from the first potential, and to conduct the electrosurgical energy through at least two of said first, second and third electrodes; and
a switch operatively coupled to at least two of said first, second and third electrodes;
wherein said second electrode is located a first distance with respect to said at least one of said interfacing surfaces from said first electrode and wherein said third electrode is located a second distance with respect to said at least one of said interfacing surfaces from said first electrode.
